# EUROPEAN PATENT APPLICATION

(11) **EP 4 140 410 A1**
(43) Date of publication of application: **01.03.2023**
(21) Application number: 22195480.3
(22) Date of filing: 15.03.2021
(51) Int. Cl.: A61B 5/391, A61B 5/11, A61B 5/00

(54) **METHOD FOR CHARACTERIZING UTERINE ACTIVITY IN A NON-PREGNANT UTERUS**

(30) Priority: 20.03.2020 EP 20382211
(62) Divisional of application: 21710999.0
(71) Applicant: Fundación Para la Investigación del Hospital Universitario y Politécnico La Fe de la Comunidad Valenciana, 46026 Valencia (ES)
(72) Inventor: ALBEROLA RUBIO, JOSÉ, VALENCIA (ES)
(74) Representative: ZBM Patents - Zea, Barlocci & Markvardsen

(57) **Abstract**

A method of characterizing a uterine peristaltic activity pattern and a computer program product are provided.

## Description

This application claims the benefit of European Patent Application 20382211.9 filed March 20, 2020 and is a divisional application of European application no. 21710999.0.

### FIELD OF THE INVENTION

The present disclosure relates to characterization of the uterine peristaltic activity of a non-pregnant uterus.

### BACKGROUND ART

Uterine peristalsis and myometrial activity are terms used to refer to contractions of the uterine muscle, which are associated with electrical activity of the muscle.

The uterine myometrium may be subject to variation in its contractile activity, which may vary from individual to individual and which may depend on factors such as the menstrual cycle, pathologies, and/or pregnancy.

Monitoring of uterine activity of a pregnant uterus is well-known, particularly as a means to monitor the progress of labour. Such monitoring is generally conducted with surface electrodes with means of a non-invasive nature, such as surface electrodes, and which monitoring generally does not negatively affect the pregnancy.

However, there are currently no known effective techniques or means for monitoring uterine peristaltic activity of a non-pregnant uterus.

US2016345817 describes a vaginal speculum including a spreading element and at least two electromyographic sensors.

It is an object of the present invention to provide methods for characterizing electrical activity information of the non-pregnant uterus.

### SUMMARY

An example probe for measuring electrical activity of the uterine muscle in a non-pregnant uterus does not fall under the scope of the claims. The probe may comprise an elongate member, a reference electrode having a rounded tip arranged at a distal end of the elongate member, a uterine internal orifice sensing electrode arranged concentrically with the reference electrode, and one or more uterine cavity wall sensing electrodes arranged between the reference electrode and the uterine internal orifice electrode. The probe may be configured such that in use (e.g., during measurement of electrical activity of the uterine muscle), the reference electrode may be in contact with the fundus of the uterus while the uterine internal orifice electrode may be in contact with the uterine internal orifice area, and the uterine cavity wall sensing electrodes are in contact with the uterine wall. The reference (or tip) electrode, uterine wall cavity electrodes, and uterine internal orifice electrode may be configured to sense electrical activity inside the uterine cavity area.

In some examples, the uterine internal orifice sensing electrode and the one or more uterine cavity sensing electrodes may comprise cylindrical electrodes (alternatively referred to as ring electrodes) and are concentrically arranged along the elongate member.

In some examples, the longitudinal separation along a longitudinal axis of the probe (or of the measurement portion of the probe) between a geometrical center point of the reference electrode and a geometrical center point of the uterine internal orifice measurement electrode may be approximately between 33.0 to 40.0 mm. In some examples the longitudinal separation may be approximately 46 mm.

In some examples, the longitudinal separation between the one or more uterine cavity wall measurement electrodes may be approximately between 4.3 to 4.7 mm. In some examples, the interelectrode distance may be approximately 4.5 mm.

In some examples, the probe may comprise a single reference electrode, a plurality of uterine cavity wall sensing electrodes, and a uterine internal orifice sensing electrode.

In some examples, the probe may comprise a single reference electrode and seven uterine cavity wall sensing electrodes.

In some examples, the probe may further comprise a cannula, wherein the cannula may be arranged such that it covers at least a portion of the probe during insertion.

In some examples, a kit may be provided comprising a probe as described herein and a cannula, wherein the cannula may be configured such that it covers or surrounds at least a portion of the probe during insertion.

A method of acquiring information of uterine muscle (peristaltic) activity in a non-pregnant uterus does not fall under the scope of the claims. The method may comprise generating a first set of analog signals based on electrical activity of uterine muscle sensed internally in a non-pregnant uterus during a luteal phase of a menstrual cycle, generating a second set of analog signals based on electrical activity of uterine muscle sensed internally in the same non-pregnant uterus during a follicular phase of the menstrual cycle, generating first digital data from the first set of generated analog signals, and generating second digital data from the second set of generated analog signals.

In an aspect, a method of characterizing a uterine peristaltic activity pattern falling under the scope of the claims comprises
- receiving first digital data representing electrical activity of a uterine muscle during a first time window,
- receiving second digital data representing electrical activity of the same uterine muscle at a second time window different than the first time window,
- obtaining at least one feature of uterine peristaltic activity based on said first digital data and second digital data.

The method may further comprise obtaining at least one feature related to peristaltic activity of the non-pregnant uterus based at least partly on said first digital data and on said second digital data.

According to some examples, said at least one feature comprises one or more of a muscle contraction frequency, peak muscle contraction intensity, mean intensity, mean contraction intensity, contraction duration, peristaltic direction, one or more features related to the differences between the peristaltic activity in the luteal and follicular phases, or any combination thereof.

The method may further comprise generating at least one pattern related to the peristaltic activity of the non-pregnant uterus based on the first digital data and second digital data corresponding to the non-pregnant uterus.

In some examples, generating at least one pattern related to peristaltic activity of the non-pregnant uterus based on the first digital data and second digital data corresponding to the non-pregnant uterus comprising generating or obtaining at least one feature based on the first digital data and second digital data corresponding to the non-pregnant uterus

In some examples, the method further comprises generating an electromyogram based on the at least one pattern of uterine peristalsis of the non-pregnant uterus.

In some examples, generating at least one pattern of uterine peristalsis comprises determining at least one feature related to uterine activity based on the first digital data and the second digital data.

The method may further comprise determining, based on the at least one pattern of uterine peristalsis, a time window during which a probability of embryo implantation may be higher in comparison to other time windows in the peristaltic activity cycle.

Alternatively or additionally, in some examples, the method may comprise determining, based on the at least one pattern of uterine peristalsis, a time window during which a probability of embryo implantation is higher than a predefined threshold.

Alternatively or additionally, in some examples, a threshold for determining the time window may be based on an upper percentile of estimated or predicted probability or likelihood, for example, the time window may be defined as days during which a probability of embryo implantation is in the upper 10th percentile in relation to other days within the cycle.

Alternatively or additionally, a time window may define, for example, a predetermined number of days for which the highest probability of embryo implantation is expected or has been determined. The predetermined number of days defining the time window, may be, for example, 3 days or 5 days.

In some examples, the method may further comprise providing at least one visual indicator to indicate said time window. For example, a visual indicator may be provided on a calendar or in a (menstrual cycle) timeline. In some examples, the method may further comprise providing at least one visual indicator in an electromyogram to indicate said time window. The electromyogram may represent a predicted or characterized peristaltic activity cycle of the individual or a portion thereof.

In some examples, the electrical activity inside a non-pregnant uterus may be sensed with a probe as described herein.

In examples, a system for measuring electrical activity of a uterine muscle in a non-pregnant uterus, not falling under the scope of the claims, may be provided. The system may comprise a probe as described herein, and a processor configured with instructions which when executed cause the processor to carry out at least one of the methods described herein, wherein the probe is operatively connected to the processor.

In another example, a non-transitory computer-readable medium may be provided, comprising instructions which when executed cause a processor to carry out at least one of the methods described herein.

In a further aspect, a computer program product is provided, comprising program instructions for causing a computing system to perform a method of characterizing a uterine peristaltic activity pattern falling under the scope of the claims. The computer program product may be embodied on a storage medium. Additionally or alternatively, the computer program product may be carried on a carrier signal.

### BRIEF DESCRIPTION OF THE DRAWINGS

Non-limiting examples of the present disclosure will be described in the following, with reference to the appended drawings, in which:
Figure 1 schematically illustrates a probe not falling within the scope of the claims;
Figure 2 schematically illustrates a probe, not falling within the scope of the claims, in a measurement position within a non-pregnant uterine cavity;
Figures 3A-3C schematically illustrates a probe not falling within the scope of the claims for sensing electrical activity in a non-pregnant uterus, wherein the elongate member is provided with a cannula;
Figure 4 schematically illustrates a system for characterizing and visualizing electrical activity in a non-pregnant uterus, not falling within the scope of the claims;
Figure 5 schematically illustrates a method of acquiring information of electrical activity in a non-pregnant uterus, not falling within the scope of the claims;
Figure 6 is an example of an electromyogram and a calculated signal for representation of peristaltic activity according to examples of the invention;
Figures 7A and 7B show an example of a mean signal frequency sensed by each of the plurality of electrodes in a probe during a measurement session, in a follicular phase and in a luteal phase, according to examples not falling within the scope of the claims.
Figure 8 shows various examples of statistical information of features derived from electrical activity measurement during luteal and follicular phases; and
Figure 9 is an example of a visual indicator for embryo implantation in an electromyogram according to examples of the disclosure.

### DETAILED DESCRIPTION OF THE EMBODIMENTS

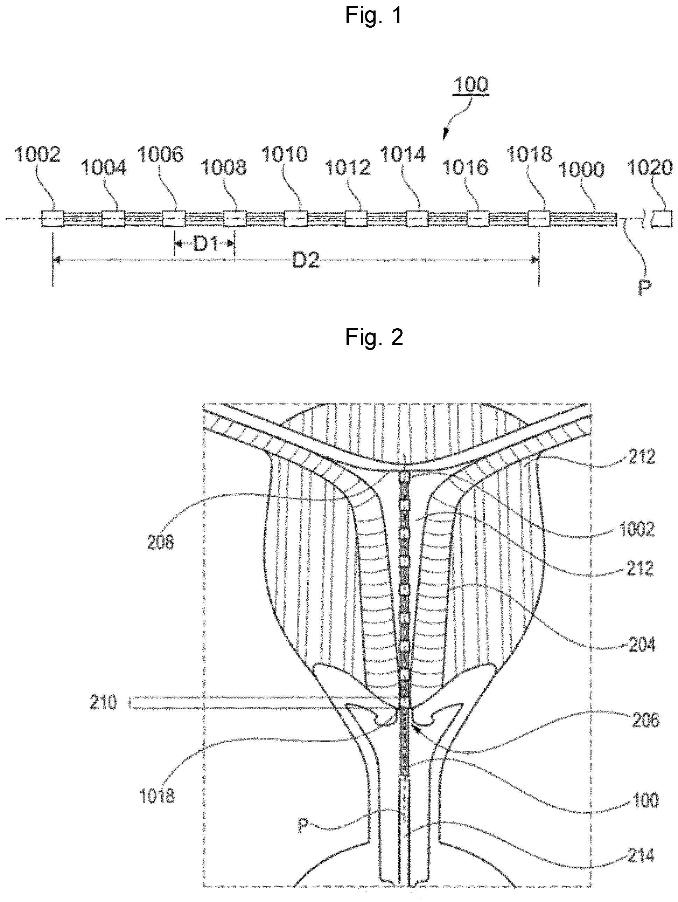

Figure 1 schematically illustrates a probe for measuring electrical activity of the uterine muscle in a nonpregnant uterine cavity. The probe 100 is a multipolar probe which comprises an elongate member 1000 and a plurality of electrodes 1002 - 1018. The electrode 1002 is a reference electrode having a substantially rounded tip arranged at a distal end of the elongate member 1000. The electrode 1018 is a uterine internal orifice measurement electrode arranged concentrically with the reference electrode 1002. A plurality of uterine cavity wall measurement electrodes 1004 - 1016 are arranged between the reference electrode and the uterine internal orifice electrode 1018.

The uterine internal orifice measurement electrode 1002 and the one or more uterine cavity measurement electrodes 1004-1016 may comprise cylindrical electrodes (also referred to as ring electrodes) concentrically arranged along the elongate member 1000.

As an example, the uterine cavity wall sensing electrodes and the uterine internal orifice electrode may have a diameter of 6F (*i.e.,* an outer diameter of 2 mm and a circumference of 6.28 mm). Other (ring) electrode diameters may also be suitable. The reference electrode (also referred herein to as the tip electrode) may have a size of 1 mm (other dimensions or sizes may also be suitable). The electrodes may comprise a metallic alloy, for example a platinum iridium platinum iridium alloy.

The elongate member 1000 preferably comprises a semi-rigid material, which may facilitate insertion and handling of the probe. Insertion of the probe into the uterine cavity may be aided by, for example, echography guiding means. The elongate member 1000 may have a curvature, for example a CS curve.

The probe 100 is configured such that during measurement of electrical activity inside the non-pregnant uterus, the reference electrode 1002 is in contact with the fundus of the uterus while the uterine internal orifice electrode 1018 is in contact with the uterine internal orifice. The electrodes sense electrical activity inside the non-pregnant uterus, a part of which corresponds to electrical activity of the uterine muscle (peristaltic activity).

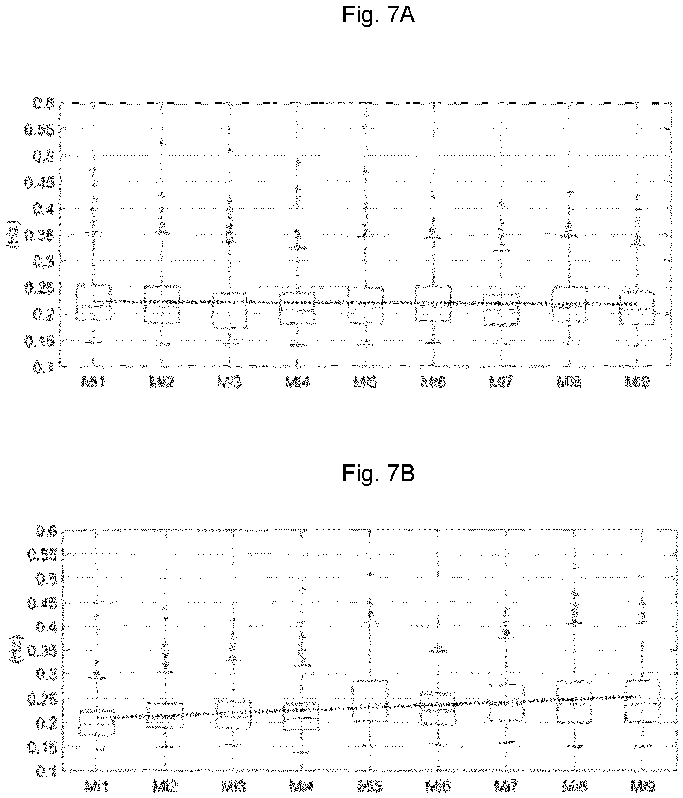

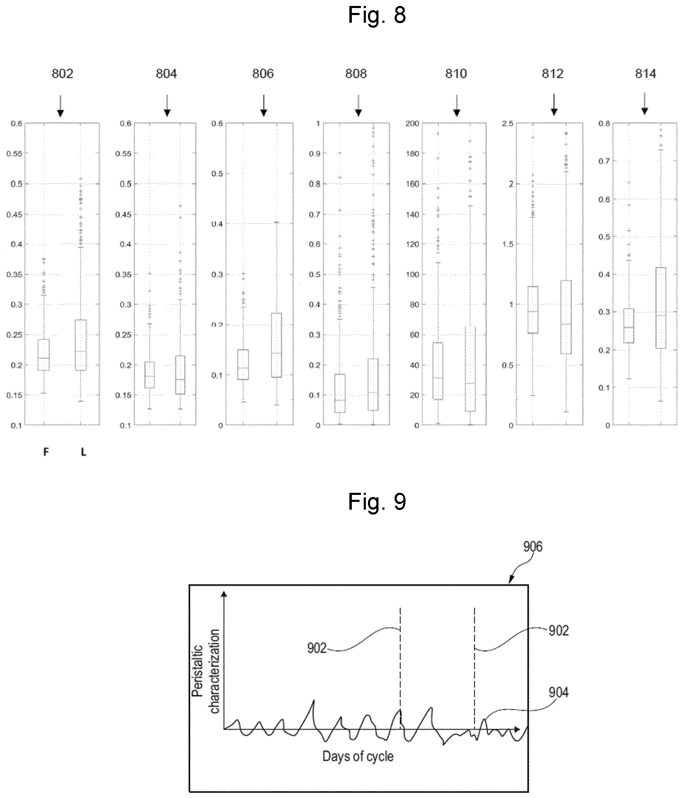

The reference electrode may have a cylindrical (or ring) portion and a rounded tip portion, which portions may be monolithically formed or separately integrated. The electrical activity sensed by each of the electrodes is output via the probe as an analog signal. Thus, the probe generates a set of analog signals during a measurement session. A probe comprising nine electrodes, as shown in Figure 1, may generate a set of nine analog signals during a measurement session. The analog signals may be transmitted to a display means, such as a monitor or screen, for visualization (which may be referred to as an electromyogram). Examples of such electromyograms are shown in Figures 7 and 8. The analog signals may also be transmitted to a processor. In order to transmit the analog signals generated by the electrodes to a processor and/or to visualization means, a connector (for example, a REDEL connector or other suitable connector) may be attached to the probe.

By having a plurality of electrodes for sensing the electrical activity throughout the extension of the uterine cavity, additional information may be derived about the peristaltic activity by correlating and/or comparing the output of the electrodes. For example, aspects such as the origin and direction of propagation of a peristaltic wave, location of peak intensity, or other directionality aspects of the electrical activity may be derived. As shown in Figures 7A and 7B, each of the plurality of electrodes may generate a different analog signal corresponding to the sensed electrical activity inside the non-pregnant uterus by the particular electrode. Figure 7A shows the analog signal frequency average sensed by each of the nine electrodes in a follicular phase. Figure 7B shows the analog signal frequency average sensed by each of the nine electrodes in a luteal phase. Differences in the mean frequency value between the luteal and follicular phases may be observed.

The longitudinal separation (D2) between a geometrical center point of the cylindrical portion of the reference (tip) electrode at a distal end of the probe 100 and a geometrical center point of the uterine internal orifice sensing electrode may be approximately between 30.0 to 42.0 mm.

In some examples, the interelectrode distance (or longitudinal separation between the geometrical center points of the nine electrodes along the longitudinal axis (P) of the probe) may be between 4.3 to 4.7 mm. In some examples, the interelectrode distance is 4.5 mm.

The probe 100 may comprise a handle portion 1020, which may be monolithically or removably integrated with the measurement portion of the probe. The handle portion 1020 may be a mere extension of the elongate member 1000 and may comprise the same material thereof. The probe may have a length (D3) of, for example, approximately between 40 cm to 90 cm such that a probe handle portion extends externally through the vaginal opening during measurement. The measurement area of the probe (i.e., the portion of the probe comprising the electrodes) may extend between, for example, 30.0 to 50.0 mm. The probe dimensions described herein may vary without departing from the scope of the invention.

The probe may further comprise a mechanism for adjustment of the distance between the reference electrode 1002 and the internal uterine orifice electrode 1008. In this manner, the probe may be adapted to a particular uterine anatomy prior to a measurement session. For example, the probe may include an extension and retraction mechanism (such as a telescopic extension mechanism) allowing for displacement along the elongate member 1000 of the uterine internal orifice electrode 1018 within a predefined extension of the elongate member 1000 (for example, 5 cm). Alternatively, the uterine internal electrode may be slidably arranged on the elongate member 1000 such that it can be displaced along an extension of the elongate member 1000 (for example along an extension of 5 cm of the elongate member 1000). Such a range or displacement distance may be selected such that the probe may be effectively utilized on substantially the global population or a particular segment thereof. Alternatively, the probe may comprise a constellation or fan configuration, wherein a plurality of elongate members comprising sensing electrodes are arranged so as to expand in a balloon-like manner inside the uterine cavity. This may advantageously enhance contact between the sensing electrodes and the uterine tissue, thereby improving sensing of the electrical activity inside the uterus.

Figure 2 schematically illustrates the probe 100 in a measurement position within a uterine cavity 202 of a non-pregnant uterus 200. The probe electrodes sense electrical activity of the myometrium or uterine muscle 212.

In order to sense electrical activity of the uterine muscle from inside the non-pregnant uterus, the probe 100 is aseptically inserted into the uterine cavity 202 of the non-pregnant uterus 200 via the vaginal opening (not shown), through the cervical cavity 214 and through the cervical opening 206. The probe is positioned such that the reference electrode 1002 (also referred as the reference pole) is in substantial contact with the upper uterine fundus area 208, while the uterine cavity wall electrodes 1004 - 1016 are positioned in substantial contact with the lateral walls (also referred to as the endometrium) 204 of the uterine cavity 202 and the uterine internal orifice electrode 1018 is in substantial contact with the uterine internal orifice area 210 (which may alternatively be referred to as the cervical opening area). In order to perform a measurement, the probe is inserted vaginally and through the cervical opening and cervical canal and is displaced inwards until the reference (or tip) electrode is in contact with the upper fundus of the non-pregnant uterus.

Although Figure 2 shows an enlarged or opened uterine cavity illustrative purposes, the lateral walls of a non-pregnant uterus are generally in substantial contact with each other. Thus, during measurement the uterine cavity wall electrodes of the probe will be in substantial contact with the uterine lateral walls, the electrodes being effectively surrounded by the lateral walls of the uterus.

At least one surface electrode (not shown) may be placed on sin surface area of the individual being measured, for example, on an internal area of the leg (thigh) of the individual being measured, in order to function as a ground (GND) electrode.

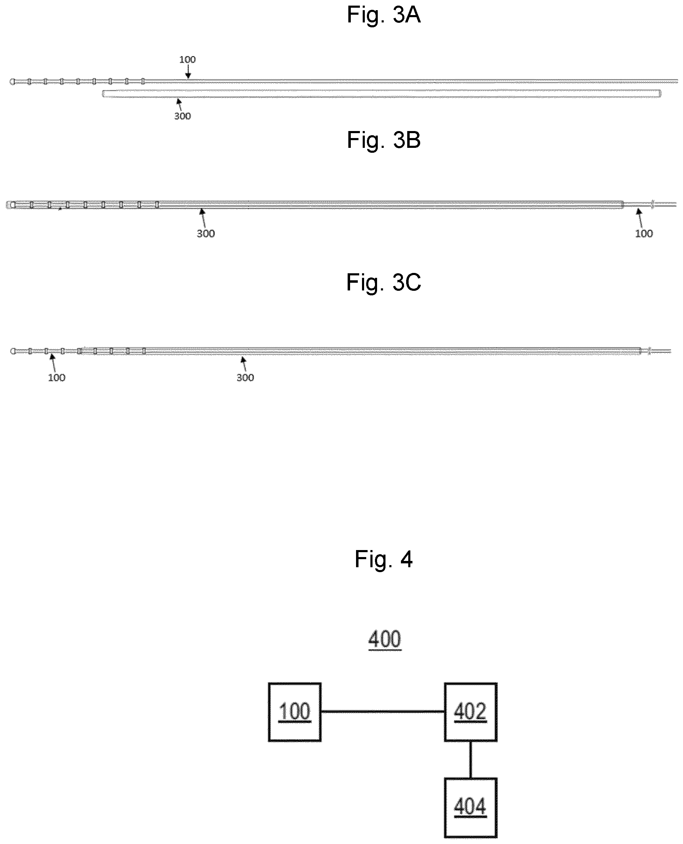

Figures 3A-3C show a probe not falling within the scope of the claims, in which a probe 100 as described is covered with a cannula 300 during part of the insertion process of the probe 100. The cannula 300 is arranged to at least cover a measurement portion 106 of the probe 100 and an extension of the probe to be inserted for measurement. The cannula 300 helps decrease the risk of infection in the individual.

As shown in Fig. 3B, in a covering position, the distal end 302 of the cannula 300 may extend beyond the distal end 108 of the probe 100.

A probe 100 covered with a cannula 300 as described herein may be inserted vaginally and displaced until the distal end 108 of the probe 100 reaches the cervical opening. At this stage, the probe 100 may be pushed along a direction (A) into the cervical canal and uterine cavity, and the cannula 300 may be removed and/or pulled outward along a direction (B) as shown in Fig. 3C.

The cannula 300 may have a length of approximately between 20 and 50 cm.

Fig. 4 shows a system 400, in which probe 100 is operatively connected to a processor 402 and visualization means 404, via a plurality of electrical connectors. Each of the plurality of connectors is associated with one of sensing electrodes (including the reference electrode), and respectively transmit the electrical analog signal generated by each of the plurality of electrodes to the processor 402 and/or visualization means 404.

The analog signals generated by the probe may be bioamplified prior to digitization and processing.

Such bioamplification may be implemented with, for example, a general purpose bioamplification system such as Grass Technologies 15LT. The bioamplification may enhance the bandwidth of interest and reduce noise. This enables the application of various filters in order to condition the electromyographic signal(s). The filters may be applied in the analog or in the digital domain, or a combination thereof.

The (filtered) signals may be digitized, for example, with an ADC (analog-to-digital converter) such as NI-6211, thereby obtaining digital data. Digitization of the (filtered) analog signals may be conducted with a sampling frequency of at least 1 KHz. Any sampling frequency at or above the Nyquist frequency will be suitable to digitize the filtered analog signals and obtain digital data therefrom. Preferably, digitization with at least 16-bit resolution is performed.

The analog (or digitized) signals may be downsampled and/or decimated (in the analog or digital domain, as applicable), such that a signal bandwidth of approximately 0.01 to 20 Hz is obtained. Alternatively, the analog (or digitized) signals may be filtered (in the analog or digital domain, as applicable) so as to obtain a signal bandwidth of approximately 0.01 to 30 Hz. Decimating and/or downsampling may comprise filtering with a bandpass filter, a low-pass filter, a high-pass filter, or any combination thereof.

The downsampled (and/or decimated) signals are subsequently filtered to obtain a frequency band between 0 to 2 Hz. It has been found that focusing on this frequency range may be advantageous for obtaining information about peristaltic activity in a non-pregnant uterus. Alternatively, the downsampled (and/or decimated) signals may be filtered to obtain a frequency band between 0 to 4 Hz. Filter(s) mentioned herein may comprise one or more of a bandpass filter, low-pass filter, high-pass filter, or any combination thereof.

The system may further comprise data storage means, such as a memory, for storing the obtained digital data. The digital data may be stored at a predefined frequency rate. For example, the received signals may be stored at least once per minute.

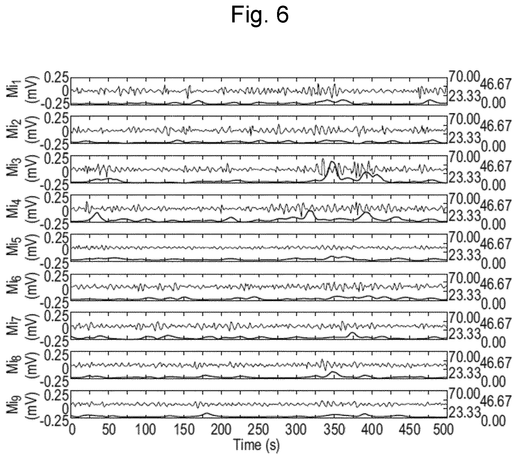

The visualization means 404 may comprise display means such as a monitor or screen. The visualization means 404 may further comprise a GUI (graphical user interface) allowing, for example, for display of each (digitized) signal in a different tab or window. As shown in Figure 6, a set of analog signals (the ones in lighter line) comprising, in this example, nine separate signals Mi₁ to Mig may be obtained during a measurement (corresponding to the electrical activity sensed inside the non-pregnant uterus 200) and be displayed in the visualization means 404. Such visualization or plotting or trace of the signal(s) may be referred to as an electromyogram or an electrohisterogram. A calculated signal (depicted by the darker or bolded line) based on the corresponding signals is also shown, which calculated signal may be used, for example, for the (visual) determination and/or detection of peristalsis or uterine contractions.

It is noted that, the systems, methods and devices described herein, may also be used for the detection of one or more of the following gynaecological conditions: miscarriages or recurrent miscarriages (i.e., spontaneous abortions), dysmenorrhea, endometriosis and / or pelvic pain since the detection of any of these gynaecological conditions may be related to abnormal patterns of uterine contractions.

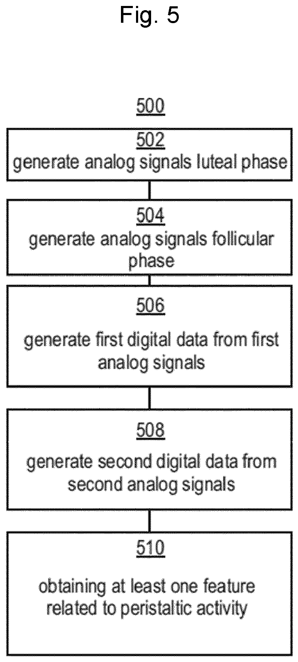

Figure 5 is a diagram of an exemplary method 500 of acquiring information of uterine muscle activity in a non-pregnant uterus not falling within the scope of the claims.

At step 502, a first set of analog signals is generated based on electrical activity of uterine muscle sensed internally in a non-pregnant uterus during a luteal phase of a menstrual cycle.

At step 504 a second set of analog signals is generated based on electrical activity of uterine muscle sensed internally in the same non-pregnant uterus during a follicular phase of the menstrual cycle.

The sequence or order of steps 502 and 504 is interchangeable. Thus, the luteal phase measurement or analog signal generation thereof may occur before or after the follicular phase measurement or analog signal generation thereof.

At step 506, generating first digital data from the first set of generated analog signals.

At step 508, generating second digital data from the second set of generated analog signals.

The sequence or order of digitization steps 506 and 508 is interchangeable, or alternatively may be performed substantially simultaneously.

In particular, the analog signals are downsampled to obtain a range of interest of 0 to 20 Hz (or alternatively of up to 30 Hz), and filtered to obtain a final band of 0 to 2 Hz. Alternatively, the signals may be downsampled to obtain a final band of 0 to 4 Hz and/or sub-bands thereof.

At step 510, at least one feature is obtained related to peristaltic activity of the non-pregnant uterus based at least partly on said first digital data and on said second digital data.

Figure 8 shows a graphical depiction of various example (statistical) features which may be obtained from the first digital data and the second digital data, corresponding to a luteal phase measurement and a follicular phase measurement. Differences in the (statistical) values of the features between the luteal and follicular phases may be observed. Some example features are mean frequency (802), frequency median (804), standard deviation (806), ratio of high and low frequencies (808), first unnormalized moment (810), RMS (812), and entropy (814). In the figure, the leftmost box for each metric represents the follicular phase value, while the rightmost box for each metric represents the luteal phase value.

The at least one feature may comprise one or more of a contraction frequency, peak contraction intensity, mean contraction intensity, median contraction intensity, median contraction duration, mean contraction duration, peak contraction duration, shortest contraction duration, lowest contraction intensity, direction of propagation of a peristaltic wave, mean direction of propagation of a peristaltic wave, median direction of propagation of a peristaltic wave, peristaltic wave origin, mean peristaltic wave origin, median peristaltic wave origin, one or more features related to the differences between the peristaltic activity in the luteal and follicular phases, entropy, intensity/amplitude RMS, first normalized moment, contraction frequency standard deviation, ratio between high and low contraction frequencies, or any combination thereof, and/or any statistical representation of any order of the aforementioned features or any combination thereof. Contraction herein refers to uterine muscle contraction (peristaltic activity of the uterus). Any of the aforementioned features, and any combination thereof may be used to detect a contraction or a contraction event in the first and second digital data. For example, threshold values for each of the aforementioned features may be established, such that, a contraction or contraction event is positively detected based on a comparison between the measured or calculated value of the feature and the threshold value established for the feature. Thus, a discrete numerical and/or categorical characterization of the peristaltic activity may be obtained.

At step 512, at least one pattern is generated related to the peristaltic activity of the non-pregnant uterus based on the first digital data and second digital data corresponding to the non-pregnant uterus. A pattern may be defined as a characterization of the peristaltic activity, which may be based on the at least one obtained feature, or may be an output of another process on the analog signal(s), for example, averaging (e.g., window-based or other type of signal averaging), on the first and second digital data. The generated pattern may comprise the complete peristaltic activity cycle (corresponding to the entire menstrual cycle) or may correspond to at least a portion thereof.

An electromyogram (or electrohisterogram) may be generated based on the at least one pattern of uterine peristalsis of the non-pregnant uterus.

A time window during which a probability of embryo implantation is highest during the peristaltic activity cycle may be determined based on the at least one pattern of uterine peristalsis. Alternatively or additionally, multiple such time windows may be determined. Visual indicators corresponding to each determined time window may be provided.

It has been found that certain part(s) of the peristaltic activity pattern may provide more favorable conditions to embryo implantation. The peristaltic activity pattern may vary from individual to individual. In each individual peristaltic activity cycle, there may be different patterns or phases thereof within the cycle which will be more conducive for embryo implantation. The one or more features (or statistical derivatives thereof) or characterized pattern derived according to the methods described herein may be used in identifying and selecting the best pattern or activity segment for embryo implantation.

A time window during which the peristaltic activity will be more conducive to embryo implantation may be determined using any number of techniques and/or criteria. For example, the method may comprise determining, based on the at least one pattern of uterine peristalsis, a time window during which a probability of embryo implantation is higher than a predefined threshold. The predefined threshold may be, for example, a minimum of 0.5 likelihood (or 50% probability) of embryo implantation.

A pattern or cycle segment during which the peristaltic activity will be more conducive to embryo implantation may be determined using any number of techniques and/or criteria. For example, the method may comprise determining, based on the at least one pattern of uterine peristalsis, a peristaltic activity pattern (or cycle) segment during which a probability of embryo implantation is higher than a predefined threshold. The predefined threshold may be, for example, a minimum of 0.5 likelihood (or 50% probability) of embryo implantation. A recommended or suggested time window for embryo implantation may be determined based on the determined pattern segment.

The probability of embryo implantation may be determined based on the first digital data and the second digital data, based on the at least one pattern of uterine peristalsis, based on the at least one feature, or any combination thereof. The various features and/or any data factored into the probability determination may be weighted accordingly, based on its determined, predicted or estimated mathematical impact on the likelihood of embryo implantation.

Alternatively or additionally, a peristaltic activity pattern segment during which the peristaltic activity is more likely to lead to embryo implantation may be determined based on the at least one pattern of uterine peristalsis. Alternatively, a time window may be determined based on one or more determined peristaltic activity features, such as frequency of contractions, directionality of contractions, or intensity of contractions, peak intensity, or statistical derivatives thereof, and/or any combination thereof. Different phases of the pattern or cycle may be identified based on the features and/or statistical information thereof. In some examples, determining at least one pattern of peristaltic activity comprises obtaining at least one feature based on the first digital data and second digital data. A recommended or suggested time window for embryo implantation may be determined based on the determined pattern segment.

Alternatively or additionally, a time window(s) during which the peristaltic activity is more likely to lead to embryo implantation may be determined based on the at least one pattern of uterine peristalsis. Alternatively, a time window may be determined based on one or more determined peristaltic activity features, such as (expected) frequency of contractions, (expected or predicted) directionality of contractions, or (expected or predicted or estimated) average intensity of contractions, (expected or predicted or estimated) peak intensity, or a combination thereof. Different phases of the pattern or cycle may be identified based on the features and/or statistical information thereof. In some examples, determining at least one pattern of peristaltic activity comprises obtaining at least one feature based on the first digital data and second digital data.

The first digital data and second digital data may be analysed to obtain time-based, frequency-based, and non-linear values such as the duration of a contractile event, mean frequency, median frequency, standard deviation of the frequency, dominant frequency, dominant frequency energy, dominant frequency normalized energy, power, low band normalized energy, high and normalized energy, normalized ratio energies, unnormalized first moment, RMS (Root Mean Square), ARV (average rectifier value), sample entropy (M=1, M = 2), spectral entropy (M = 1 low band, M = 2 high band, M = 1 high band, M = 2 low band), sample kurtosis, sample asymmetry, spectral asymmetry, peak-to-peak voltage, dominant and mean radiofrequency, dominant and mean radio frequency wherein the normalized energy is factored in, dominant and mean radio frequency wherein the area under the curve is factored in, dominant and mean radio frequency wherein the sample kurtosis is factored in, dominant and mean radio frequency wherein the sample asymmetry is factored in, log detector, wave length, variance, EHG integration, mean absolute value, spectral entropy, Teaguer, and Lanipuv, among other values. Such statistical information may be used as reference to compare obtained measurements and detect pathologies or estimate optimal embryo implantation time windows or parts of the menstrual cycle in a non-pregnant individual. Additionally, models for such predictions and determinations may be created or based on SVM (support vector machines), auto-regression models, machine learning, artificial neural networks (ANN), and linear and non-linear regressions and correlations.

Once a time window has been determined, for example, as a date range, such information may be provided in any number of ways. For example, a visual indicator may be provided in a calendar and/or in an electromyogram (representing, for example, the entire uterine peristaltic/menstrual cycle) to indicate said time window. Figure 9 shows an example of a visual indicator, in which a visual indicator 902 is provided on the electromyogram or characterization/pattern depiction 904 (or portion thereof), on display means 906. The visual indicator 902 demarcates a time window within the menstrual cycle. In this example, the peristaltic activity characterization (for example, pattern) is plotted on the vertical axis with respect to the enumerated days of the menstrual cycle which are marked on the horizontal axis.

A time window may be mapped onto a timeline (for example, a timeline indicating a menstrual cycle in days) or calendar (for example, a Gregorian calendar), wherein the time window may comprise a demarcation of a date range comprising, for example, a specific day or a range of days. Other data may be factored in determining the time window, such as, expected or predicted uterine temperature (value and/or variations thereof) on or throughout the particular day (or range of days) of the cycle. Each factor may be weighted accordingly on the basis of its measured or predicted impact on embryo implantation.

It may be additionally determined where in the menstrual cycle a particular peristaltic pattern (or part thereof) lies with respect to the calendar or timeline.

Once a time window has been determined and selected, an embryo(s) implantation procedure may be coordinated accordingly. In this manner, odds of successful embryo implantation may be optimized.

In order to sense electrical activity inside the non-pregnant uterus, a probe as described herein may be used. Alternatively, the electrical activity may be sensed directly inside the non-pregnant uterus using other suitable electrical activity sensing means. The set of analog signals corresponds to a measurement session of an individual. The measurement or information acquirement session may last approximately 20 to 25 minutes. In some examples, a measurement session may last longer, for example between 25 and 45 minutes, or may be shorter, for example, under 20 minutes. A probe as shown in any of Figs. 1-3C may be used to generate the first and second sets of analog signals. In order to generate the (first and second sets of) analog signals, the electrical activity may be measured during, for example, a time span of 45 minutes (both in a luteal phase, and a follicular phase of the menstrual cycle). The peristaltic activity may be monitored in the visualization means, and the measurement session may last as long as deemed necessary. A longer measurement session may be conducted when the peristaltic activity appears to be irregular or highly irregular.

### CLAUSES

For reasons of completeness, various example features are set out in the following numbered clauses:
Clause 1.Probe for measuring uterine muscle activity in a non-pregnant uterus, the probe comprising:
   - an elongate member,
   - a reference electrode having a rounded tip arranged at a distal end of the elongate member,
   - a uterine internal orifice sensing electrode arranged concentrically with the reference electrode, and
   - one or more uterine cavity wall sensing electrodes arranged between the reference electrode and the uterine internal orifice sensing electrode,
   - wherein the electrodes are separated by an interelectrode distance (D1) along the longitudinal axis (P) of the probe, and
   wherein the probe is configured such that, in use, the reference electrode is in contact with a fundus of the uterus while the uterine internal orifice electrode is in contact with a uterine internal orifice.
Clause 2.Probe according to Clause 1, wherein the uterine internal orifice measurement electrode and the one or more uterine cavity wall sensing electrodes comprise cylindrical electrodes and are concentrically arranged along the elongate member.
Clause 3.Probe according to any of the preceding Clauses, wherein the longitudinal separation between a geometrical center point of the reference electrode and a geometrical center point of the uterine internal orifice sensing electrode is between 33.0 to 40.0 mm.
Clause 4.Probe according to any of the preceding Clauses, wherein the longitudinal separation (D1) between the one or more sensing electrodes is between 4.3 to 4.7 mm.
Clause 5.Probe according to any of the preceding Clauses, wherein the probe comprises a single reference electrode and seven uterine cavity wall sensing electrodes.
Clause 6.Probe according to any of the preceding Clauses, further comprising a cannula, wherein the elongate member is arranged for displacement within the cannula, and wherein the probe is configured such that cannula is covers a portion of the elongate member during insertion of the probe.
Clause 7. A kit comprising a probe according to any of Clauses 1 to 5 and a cannula, wherein the cannula is configured to cover at least a portion of the elongate member/the probe during insertion of the probe.
Clause 8. Method of characterizing a uterine peristaltic activity pattern comprising
   - receiving first digital data representing electrical activity of a uterine muscle during a first time window,
   - receiving second digital data representing electrical activity of the same uterine muscle at a second time window different than the first time window,
   - obtaining at least one feature of uterine peristaltic activity based on said first digital data and second digital data.
Clause 9. Method of Clause 8, wherein the first time window is during a luteal phase of a menstrual cycle associated with the uterine muscle, and wherein the second time window is during a follicular phase of said menstrual cycle, or vice versa.
Clause 10. The method of Clause 8, further comprising generating at least one pattern based on the at least one feature.
Clause 11. Method of acquiring information of uterine peristaltic activity in a non-pregnant uterus, comprising:
   - placing electrical activity sensing means inside the uterine cavity of a non-pregnant uterus to sense the electrical activity of the uterine muscle during a first time window, thereby generating at least one first analog signal,
   - placing electrical activity sensing means inside the uterine cavity of a non-pregnant uterus to sense the electrical activity of the uterine muscle during a second time window, thereby generating at least one second analog signal,
   - generating first digital data from the at least one first analog signal,
   - generating second digital data from the at least one second analog signal, and
   - characterizing the uterine activity of the non-pregnant uterus by obtaining at least one feature based on the first and second digital data.
Clause 12. Method of Clause 11, wherein the first time window falls within a luteal phase of a menstrual cycle associated with the uterine muscle, and wherein the second time window falls within a follicular phase of said menstrual cycle, or vice versa.
Clause 13. Method of Clause 11 or Clause 12, further comprising generating at least one pattern based on the at least one feature.
Clause 14. Method of acquiring information of uterine peristaltic activity in a non-pregnant uterus comprising:
   - inserting a probe inside the uterine cavity via the vaginal opening,
   - generating first set of analog signals during a first time window of a first phase of a menstrual cycle,
   - generating a second set of analog signals during a second time window of a second phase of a menstrual cycle,
   - generating first digital data from the first set of analog signals,
   - generating second digital data from the second set of analog signals,
   - obtaining at least one feature of uterine peristaltic activity based on said first digital data and second digital data,

   wherein the probe comprises an elongate member,
   oa reference electrode having a rounded tip arranged at a distal end of the elongate member,
   oa uterine internal orifice sensing electrode arranged concentrically with the reference electrode, and
   oone or more uterine cavity wall sensing electrodes arranged between the reference electrode and the uterine internal orifice sensing electrode,
   owherein the electrodes are separated by an interelectrode distance (D1) along the longitudinal axis (P) of the probe, and
   wherein the probe is configured such that, in use, the reference electrode is in contact with a fundus of the uterus while the uterine internal orifice electrode is in contact with a uterine internal orifice.
Clause 15. Method of Clause 14, wherein the first time window occurs during a luteal phase of a menstrual cycle associated with the uterine muscle, and wherein the second time window occurs during a follicular phase of said menstrual cycle, or vice versa.
Clause 16. Method of Clause 14 or Clause 15, further comprising generating at least one pattern based on the at least one feature.
Clause 17. Method of determining an optimal embryo implantation time window, comprising the steps of
   - placing electrical activity sensing means inside the uterine cavity of a non-pregnant uterus to sense the electrical activity of the uterine muscle during a first time window,
   - placing electrical activity sensing means inside the uterine cavity of a non-pregnant uterus to sense the electrical activity of the uterine muscle during a second time window, thereby generating at least one second analog signal,
   - generating first digital data from the at least one first analog signal,
   - generating second digital data from the at least one second analog signal, and
   - determining, based on the first digital data and the second digital data, a time window during which a probability of embryo implantation is higher than a predefined threshold.
Clause 18. Method of Clause 17, wherein determining a time window based on the first digital data and the second digital data comprises obtaining at least one feature based on the first and second digital data.
Clause 19. Method of Clause 18 or Clause 17, wherein determining a time window based on the first digital data and the second digital data comprises generating at least one pattern based on the first and second digital data.
Clause 20. Method of Clause 19, further comprising providing at least one visual indicator on a visual representation of at least a portion of the at least one pattern to indicate said time window.
Clause 21. Method of any of Clauses 17 to 20, wherein placing electrical activity sensing means inside the uterine cavity of a non-pregnant uterus to sense the electrical activity of the uterine muscle during a first time window and during a second time window comprises inserting a probe according to any of Clauses 1 to 7 into the uterine cavity via a vaginal opening.
Clause 22. Method of characterizing uterine activity and/or generating an electromyogram, the method comprising:
   - inserting a probe according to any of Clauses 1 to 7 via a vaginal opening to internally sense the electrical activity of a non-pregnant uterus of uterine muscle during a luteal phase of a menstrual cycle to generate a first set of analog signals based on the sensed electrical activity,
   - inserting a probe according to any of Clauses 1 to 7 via a vaginal opening to internally sense the electrical activity of a non-pregnant uterus of uterine muscle during a follicular phase of a menstrual cycle to generate a second set of analog signals based on the sensed electrical activity,
   - generating first digital data from the first set of generated analog signals, and
   - generating second digital data from the second set of generated analog signals.
Clause 23. Method of Clause 22, further comprising generating at least one pattern and/or obtaining at least one feature based on the first digital data and the second digital data.
Clause 24. Method according to Clause 23, further comprising diagnosing a peristaltic uterine anomaly, pathology or associated pathology based on the at least one pattern or the at least one feature.
Clause 25. Method of Clause 23, further comprising repeating the steps of Clause 22, thereby obtaining a plurality of features and/or patterns associated with electrical activity of different non-pregnant uteri, obtaining statistical information of the plurality of features and/or patterns.
Clause 26. Computer readable medium encoded with instructions which cause a processor to execute at least one of the methods according to any of Clauses 8 to 25.
Clause 27. A computer program product comprising program instructions for causing a computing system to perform a method according to any of Clauses 8 to 25.
Clause 28. A computer program product according to Clause 27, embodied on a storage medium.

## Claims

1. Method of characterizing a uterine peristaltic activity pattern, the method comprising
- receiving first digital data representing electrical activity of a uterine muscle during a first time window,
- receiving second digital data representing electrical activity of the same uterine muscle at a second time window different than the first time window,
- obtaining at least one feature of uterine peristaltic activity based on said first digital data and second digital data.

2. Method of claim 1, wherein the first time window is during a luteal phase of a menstrual cycle associated with the uterine muscle, and wherein the second time window is during a follicular phase of said menstrual cycle, or vice versa.

3. The method of claim 1 or 2, further comprising generating at least one pattern based on the at least one feature.

4. Method according to any of claims 1 to 3, wherein said at least one feature comprises one or more of a muscle contraction frequency, peak muscle contraction intensity, mean intensity, mean contraction intensity, contraction duration, peristaltic direction, one or more features related to the differences between the peristaltic activity in the luteal and follicular phases, or any combination thereof.

5. Method according to any of claims 1 to 4, further comprising generating at least one pattern related to the peristaltic activity of the non-pregnant uterus based on the first digital data and second digital data corresponding to the non-pregnant uterus.

6. Method according to claim 5, further comprising generating an electromyogram based on the at least one pattern of uterine peristalsis of the non-pregnant uterus.

7. Method according to claim 6, further comprising determining, based on the at least one pattern of uterine peristalsis, a time window during which a probability of embryo implantation is higher than a predefined threshold.

8. Method according to claim 7, further comprising providing at least one visual indicator in said electromyogram to indicate said time window.

9. Computer program product comprising program instructions for causing a computing system to perform a method according to any of the claims 1 to 8.
